# EUROPÄISCHE PATENTANMELDUNG

(11) **EP 4 019 057 A1**
(43) Veröffentlichungstag der Anmeldung: **29.06.2022**
(21) Anmeldenummer: 22157269.6
(22) Anmeldetag: 02.06.2017
(51) Int. Cl.: A61L 27/10, A61L 27/56, C04B 35/48

(54) **KNOCHENERSATZMATERIAL AUS ZIRKONDIOXIDKERAMIK**

(30) Priorität: 09.06.2016 DE 102016110622; 22.06.2016 DE 102016111431
(62) Teilanmeldung aus: 17727880.1
(71) Anmelder: Gahlert, Michael, 81925 München (DE); Apano GmbH, 04683 Naunhof (DE)
(72) Erfinder: GAHLERT, Michael, 81925 München (DE); GAHLERT, Stefan, 70619 Stuttgart (DE)
(74) Vertreter: Witte, Weller & Partner Patentanwälte mbB

(57) **Zusammenfassung**

Es wird ein Verfahren zur Herstellung eines Knochenersatzmaterials angegeben, das aus einer Zirkondioxidkeramik besteht, die porös sein kann, insbesondere eine offene Porosität aufweisen kann. Das Knochenersatzmaterial kann in Partikelform oder in Blockform verwendet werden.

## Beschreibung

Die Erfindung betrifft ein Knochenersatzmaterial, sowie ein Verfahren zur Herstellung eines solchen.

In der zahnärztlichen Implantologie werden kompromittierte anatomische Knochenstrukturen häufig mit zusätzlichen Knochenaufbaumaßnahmen ergänzt. Neben der Möglichkeit, körpereigenen, vitalen Knochen einer Spenderregion in die Empfängerregion zu transferieren (autologer Knochen), gibt es auch die Möglichkeit, ein Knochenersatzmaterial (KEM) zu verwenden.

Es wird dabei zwischen allogenen, alloplastischen und xenogenen KEM unterschieden. Unter allogenem KEM versteht man Knochen von anderen Menschen, der labortechnisch durch Entkalkung oder Gefriertrocknung aufbereitet wurde. Das alloplastische KEM bezeichnet synthetisch hergestelltes KEM wie zum Beispiel Hydroxylapatit, während man xenogenes KEM aus Rinderknochen, Algen oder Korallen herstellt. Bis auf das körpereigene, autologe KEM, welches die Knochenbildung anregt und als osseoinduktiv bezeichnet wird, sind alle anderen KEM osseokonduktiv. Darunter versteht man eine Leitschienenfunktion des KEM, welches ein Einsprossen von ortsständigen Kieferknochen, dem sogenannten Lagergewebe, ermöglichen soll.

Biokompatible KEM sind in der dentalen Implantologie weltweit anerkannt und in zahlreichen Studien evidenzbasiert wissenschaftlich untersucht.

Alloplastische KEM sind im Stand der Technik seit Jahrzehnten in Form diverser anorganischer KEM bekannt. Meist enthalten sie Hydroxylapatit und/oder Calciumphosphat (vgl. z.B. DE 100 60 036 C1, EP 0 006 544 A1). Teilweise wird auch Kalziumphosphat verwendet, das zu einer porösen Keramik verarbeitet wird, die auch partikelförmig vorliegen kann (vgl. DE 102 58 773 A1).

Teilweise werden dem anorganischen Knochenersatzmaterial, wie etwa Hydroxylapatit, auch organische Bestandteile beigemischt, wie etwa Kollagen, um die Osseokonduktivität zu verbessern (vgl. z.B. EP 2 826 495 A1).

Grundsätzlich besteht bei anorganischen Knochenersatzmaterialien immer das Problem, dass diese synthetisch hergestellten KEM die Knochenbildung nicht selbst anregen, sondern lediglich eine gewisse Leitschienenfunktion für die Knochenbildung bereitstellen können.

Vor diesem Hintergrund liegt der Erfindung die Aufgabe zu Grunde, ein Knochenersatzmaterial auf anorganischer Basis bereit zu stellen, das sehr gut körperverträglich ist, das eine möglichst gute Osseokonduktivität aufweist und das insbesondere in der dentalen Praxis verwendet werden kann, um in möglichst kurzer Einheilzeit eine dauerhafte Knochenbildung bzw. Osseointegration zu gewährleisten, die insbesondere zum Setzen von Implantaten geeignet ist. Ferner soll ein Verfahren zur Herstellung eines solchen Knochenersatzmaterials angegeben werden.

Diese Aufgabe wird durch eine Zirkondioxidkeramik als KEM gelöst, die vorzugsweise in Partikelform verwendet wird.

Die Aufgabe der Erfindung wird auf diese Weise vollständig gelöst.

Zirkondioxidkeramik zeichnet sich durch eine exzellente Biokompatibilität und eine vollständige Allergiefreiheit aus. Auch ohne aufwändige Zusatzmaßnahmen, wie eine Beimischung von künstlichen organischen oder tierischen Zusatzmaterialien kann eine gute Osseokonduktivität erreicht werden.

Vorzugsweise wird das KEM in Partikelform verwendet, vorzugsweise mit einer Partikelgröße im Bereich von 0,1 bis 6 mm, weiter bevorzugt im Bereich von 0,2 bis 4 mm.

Dies erlaubt eine unmittelbare Verwendung in der zahnärztlichen Implantologie, um einen individuellen Knochenaufbau in Defektbereichen zu ermöglichen.

Vorzugsweise ist das KEM makroporös mit einer offenen Porosität.

Es kann sich hierbei etwa um Makroporen mit einem mittleren Porendurchmesser im Bereich von 10 Mikrometer bis 500 Mikrometer, vorzugsweise im Bereich von 50 Mikrometer bis 300 Mikrometer, weiter bevorzugt im Bereich von 60 bis 250 Mikrometer handeln.

Durch eine solche offene Porosität wird die Osseokonduktivität deutlich verbessert. Die untereinander verbundenen Poren erlauben das Einwachsen von Blutzellen und Proteinen und erlauben so eine verbesserte Osseointegration.

Gemäß einem weiteren Merkmal der Erfindung ist das KEM mikroporös, vorzugsweise mit einer offenen Porosität.

Hierbei können die Mikroporen etwa einen mittleren Porendurchmesser von weniger als 10 Mikrometer aufweisen, vorzugsweise von weniger als 1 Mikrometer, vorzugsweise von mindestens 5 Nanometer aufweisen.

Durch eine solche Mikrostruktur ergibt sich eine Kapillarwirkung, die eine Flüssigkeitsaufnahme unterstützt. Auch hierdurch wird also eine Verbesserung der Osseokonduktion unterstützt.

Gemäß einem weiteren Merkmal der Erfindung weist das KEM eine bimodale Porosität mit Makroporen und Mikroporen auf.

Durch die Kombination der offenen Makroporosität mit der offenen Mikroporosität können so beide vorteilhaften Eigenschaften miteinander kombiniert werden. Insgesamt ergibt sich eine weiter verbesserte Osseokonduktion.

Soweit das KEM partikelförmig verwendet wird, ist in Abhängigkeit von der Partikelgröße bei sehr kleinen Partikeln entweder eine massive Ausführung bevorzugt oder aber bei größeren Partikeln gleichfalls eine makroporöse und/oder mikroporöse Ausführung, wie oben beschrieben.

Bei Partikelgrößen ab etwa 1 bis 2 Millimeter Durchmesser ist eine poröse Ausführung bevorzugt, um die Osseointegration zu verbessern.

Bei kleineren Partikelgrößen wird aus Gründen der Stabilität auf eine Makroporosität verzichtet. Allerdings kann eine Mikroporosität zusätzlich die Osseointegration verbessern.

Dagegen wird blockförmiges KEM, das in beliebiger Form hergestellt sein kann, bevorzugt mit offener Makroporosität und ggf. auch offener Mikroporosität verwendet.

Gemäß einem weiteren Merkmal der Erfindung besteht das KEM aus tetragonalem, polykristallinem Zirkondioxid (TZP). Dieses kann etwa mittels Y₂O₃ stabilisiert sein, beispielsweise durch Zugabe von 3 Gew.-% Y₂O₃.

Mit TZP werden höchste Festigkeiten erzielt. Grundsätzlich geeignet als KEM ist allerdings auch mit MgO teilstabilisiertes Zirkondioxid (PSZ).

Gemäß einem weiteren Merkmal der Erfindung liegt die Porosität im Bereich von 0,1 bis 90 %, vorzugsweise im Bereich von 1 bis 50 %, weiter bevorzugt im Bereich von 5 bis 30 %.

Mit einer derartigen Porosität wird eine besonders gute Osseokonduktion und Osseointegration erzielt.

Gemäß einem weiteren Merkmal der Erfindung wird das KEM in Blockform, vorzugsweise makroporös mit offener Porosität, verwendet.

Eine Verwendung in Blockform ermöglicht einen Knochenaufbau von größeren Bereichen. Hierzu werden vorzugsweise verschiedene vorgefertigte Abmessungen bereitgehalten.

Soweit nicht dentale Anwendungen betroffen sind, etwa bei der Rekonstruktion von diversen Knochenfrakturen, kann das KEM auch in speziellen Formen hergestellt werden, die ggf. auch patentientenspezifisch angepasst sein können.

Gemäß einem weiteren Merkmal der Erfindung weist das KEM eine geätzte Oberfläche auf.

Durch eine Ätzbehandlung, etwa mittels Flusssäure, der äußeren Oberfläche und der inneren Oberflächen infolge der offenen Porosität wird die Osseoinduktion weiter verbessert.

Die Aufgabe der Erfindung wird ferner durch ein Verfahren zum Herstellen eines porösen Knochenersatzmaterials aus einer Zirkondioxidkeramik gelöst, bei dem Zirkondioxidpulver mit organischen Zusätzen, insbesondere in Form von Kurzfasern, dreidimensionalen Strukturen, etwa in Form eines Gewebes oder Gewirkes, und/oder in Form von Pulver, vorzugsweise aus Kunststoff, versetzt wird, vorzugsweise unter Zugabe von Bindemitteln, zu einem Precurser-Körper geformt wird, und anschließend gesintert wird.

Auf diese Weise kann ein makroporöser Körper mit einer offenen Porosität erzeugt werden. Da sich die Kurzfasern bzw. die dreidimensionale Struktur oder das Pulver aus organischem Material beim Aufheizen vollständig zersetzen, kann durch die so frei werdenden Gase eine offene Porosität erzeugt werden. Gesteuert wird dies insbesondere durch den Anteil der zugesetzten Fasern, sowie deren Durchmesser und Länge, bzw. Gestaltung der dreidimensionalen Struktur, die Partikelgröße des organischen Pulvers, die Partikelgröße des Zirkondioxidpulvers und das verwendete Temperaturprogramm.

Eine bimodale Porosität kann hierbei erzeugt werden, indem der Sintervorgang gesteuert beendet wird, bevor die beim Sintern normalerweise anfangs vorhandenen Mikroporen vollständig auswachsen. Alternativ können auch organische Zusätze mit bimodaler Größenverteilung zugesetzt werden.

Soweit eine dreidimensionale Struktur etwa in Form eines Gewebes oder Gewirkes verwendet wird, so kann diese in geeigneter Weise dimensioniert werden, um eine möglichst homogene offene Porosität beim späteren Brennvorgang zu erzeugen. Auch eine Erzeugung einer Porosität mit Textur ist möglich.

Zur Herstellung einer homogenen Mischung der Komponenten ist ein Pelletieren in einem Pelletiergefäß, also auf einem drehbar angetriebenen Pelletierteller oder in einer drehbar angetriebenen Trommel besonders geeignet.

Gemäß einem weiteren Merkmal der Erfindung erfolgt die Formgebung durch Pressen, vorzugsweise uniaxiales oder isostatisches Pressen, durch Schlickergießen oder Schleudergießen mit anschließenden Trocknen, oder durch ein anders pulvertechnologisches Verfahren.

Auch hierbei werden vorzugsweise organische Zusätze, insbesondere Kurzfasern oder dreidimensionale Strukturen aus Kunststoff, mit geeignetem Durchmesser und geeigneter Länge zugesetzt, die sich beim späteren Sintern zersetzen, um so eine makroporöse Struktur zu erzeugen.

Gemäß einem weiteren Merkmal der Erfindung wird ein poröses KEM aus einer Zirkondioxidkeramik hergestellt, indem ein offenporiger Kunststoffschaum unter Entlüftung in einen Schlicker mit Zirkondioxidpulver getaucht wird, anschließend aus dem Schlicker entfernt, getrocknet und gesintert wird.

Auch auf diese Weise lässt sich ein makroporöses KEM aus Zirkondioxid herstellen. Dieses Verfahren ist im Zusammenhang mit der Herstellung von porösen Keramikkörpern als sog. Replikaverfahren bekannt.

Gemäß einem weiteren Merkmal der Erfindung wird das erhaltene Material nach dem Sintern zu Partikeln gemahlen.

Gemäß einem weiteren Merkmal der Erfindung wird mikroskaliges Zirkondioxidpulver mit einer mittleren spezifischen Oberfläche im Bereich von 5 bis 100 m²/g verwendet.

Gemäß einem weiteren Merkmal der Erfindung erfolgt das Sintern im Bereich von 850 °C bis 1750 °C, vorzugsweise im Bereich von 850 °C bis 1550 °C, weiter bevorzugt im Bereich von 900 °C bis 1350 °C.

Die verwendete Sintertemperatur ist insbesondere von der Pulverbeschaffenheit des Zirkondioxidpulvers, insbesondere von der mittleren spezifischen Oberfläche oder der mittleren Partikelgröße abhängig. Je größer die mittlere spezifische Oberfläche, um so geringer ist in der Regel die Sintertemperatur.

Mit Pulvern im Bereich von etwa 20 bis 50 m²/g liegen die Sintertemperaturen meist im Bereich von etwa 1250 °C bis 1350 °C.

Soweit ein Vorsintern zu einem Grünkörper erfolgt, der im Grünzustand mechanisch bearbeitet werden kann, um eine bestimmte Form und Größe herzustellen, liegt die Vorsintertemperatur etwa 100 bis 500 K unterhalb der Temperatur zum Fertigsintern.

### BEISPIELE

### Beispiel 1

Kommerziell erhältliches TZP-Pulver (mit 3 Gew.-% Y₂O₃ stabilisiert) mit einer mittleren spezifischen Oberfläche von 50 m²/g wird mit 20 Gew.-% Kurzfasern aus Polyvinychlorid (PVC) mit einem mittleren Durchmesser von etwa 50 Mikrometer und einer mittleren Länge von 500 Mikrometer unter Zusatz eines Bindemittels (1 Gew.-% Isopropanol) gemischt, so dass die Kurzfasern statistisch verteilt sind. Danach erfolgt ein uniaxiales Pressen unter Verwendung einer geeigneten Stahlmatrize (Druck von beispielsweise 20 bis 50 bar).

Anschließend erfolgt eine Aufheizung auf etwa 1350 °C mit etwa 50 K/min und ein Halten bei 1350 °C für etwa 30 bis 120 Minuten, danach Abkühlung durch Abschalten.

Auf diese Weise wird ein makroporöser Block aus TZP-Keramik mit einer offenen Porosität erhalten. Das so erhaltene Material kann mittels einer Kugelmühle zu porösen Partikeln vermahlen werden und dann für eine gewünschte Partikelgrößenverteilung entsprechend ausgesiebt werden.

Anstelle einer uniaxialen Pressung kann das Pulvergemisch auch isostatisch gepresst werden.

### Beispiel 2

Zur Herstellung von pulverförmigem KEM, das nicht porös ist, wird kommerziell erhältliches Zirkondioxid zur gewünschten Partikelgrößenverteilung gemahlen und anschließend ausgesiebt. Dies ist insbesondere für kleinere Partikelgrößen etwa kleiner als 1 bis 2 Millimeter Durchmesser, von Interesse.

### Beispiel 3

Das Material gemäß Beispiel 1 wird nicht vermahlen, sondern unmittelbar in Blockform verwendet. Es kann hierzu durch mechanische Bearbeitung mit Diamantwerkzeugen in die gewünschte Größe und/oder Form gebracht werden.

### Beispiel 4

Kommerziell erhältliches TZP-Pulver (mit 3 Gew.-% Y₂O₃ stabilisiert) mit einer mittleren spezifischen Oberfläche von 50 m²/g wird mit 5 Gew.-% mit 10 Gew.-% Kurzfasern aus Polyvinychlorid (PVC) mit einem mittleren Durchmesser von etwa 50 Mikrometer und einer mittleren Länge von 500 Mikrometer unter Zusatz eines Bindemittels (1 Gew.-% Isopropanol) gemischt und dann isostatisch gepresst (Druck von beispielsweise 500 bis 1000 bar).

Anschließend erfolgt eine Aufheizung auf etwa 900 °C mit etwa 50 K/min und ein Halten für etwa 15 bis 30 Minuten, danach Abkühlung durch Abschalten. Es wird ein makroporöser Grünkörper erhalten, der im Grünzustand mechanisch bearbeitet werden kann, etwa mittels einer automatisch gesteuerten Fräseinrichtung. Dabei wird das Schrumpfungsmaß für den späteren Sinterprozess berücksichtigt.

Schließlich erfolgt ein Fertigsintern bei etwa 1300 °C (Aufheizen mit etwa 50 K/min und dann Halten bei 1300 °C etwa 15 bis 60 Minuten). Es ergibt sich ein makroporöser Zirkondioxidkörper mit offener Porosität.

### Beispiel 5

Ein offenporiger Kunststoffschaum wird in einen Schlicker mit Zirkonoxidpulver gemäß Beispiel 1 getaucht, mit dem Schlicker getränkt, anschließend getrocknet (60 Minuten bei 90 °C), dann gemäß Beispiel 1 gesintert. Es ergibt sich eine offenporige Zirkonoxidkeramik.

### Beispiel 6

Als Nachbehandlung wird bei den Beispielen 1 bis 5 eine Ätzbehandlung durch Eintauchen in Flusssäure durchgeführt, in dem das fertig gesinterte und ggf. gemahlene KEM beispielsweise in 40%-ige Flusssäure getaucht wird und 5 bis 60 Minuten geätzt wird, vorzugsweise bei erhöhter Temperatur von z.B. 60 bis 70 °C.

## Patentansprüche

1. Verfahren zum Herstellen eines porösen Knochenersatzmaterials aus einer Zirkondioxidkeramik, bei dem Zirkondioxidpulver mit organischen Zusätzen, insbesondere in Form von Kurzfasern, dreidimensionalen Strukturen, etwa in Form eines Gewebes oder Gewirkes, und/oder in Form von Pulver, vorzugsweise aus Kunststoff, versetzt wird, vorzugsweise unter Zugabe von Bindemitteln, zu einem Precurser-Körper geformt wird, und anschließend gesintert wird.

2. Verfahren nach Anspruch 1, bei dem Kurzfasern mit einem mittleren Durchmesser von im Bereich von 10 Mikrometer bis 500 Mikrometer, vorzugsweise im Bereich von 50 Mikrometer bis 300 Mikrometer, weiter bevorzugt im Bereich von 60 bis 250 Mikrometer verwendet werden.

3. Verfahren nach Anspruch 1 oder 2, bei dem Kurzfasern mit einer mittleren Länge von 10 Mikrometer bis 20 Millimeter, vorzugsweise von 50 Mikrometer bis 2000 Mikrometer, besonders bevorzugt von 100 Mikrometer bis 1000 Mikrometer verwendet werden.

4. Verfahren nach einem der Ansprüche 1 bis 4, bei dem Kurzfasern mit bimodaler Durchmesserverteilung zugesetzt werden.

5. Knochenersatzmaterial aus einer Zirkondioxidkeramik, vorzugsweise in Partikelform.

6. Knochenersatzmaterial nach Anspruch 5, mit einer Partikelgröße im Bereich von 0,1 bis 6 mm, bevorzugt im Bereich von 0,2 bis 4 mm.

7. Knochenersatzmaterial nach Anspruch 5 oder 6, das makroporös mit einer offenen Porosität ist.

8. Knochenersatzmaterial nach Anspruch 7, das Makroporen mit einem mittleren Porendurchmesser im Bereich von 10 Mikrometer bis 500 Mikrometer, vorzugsweise im Bereich von 50 Mikrometer bis 300 Mikrometer, weiter bevorzugt im Bereich von 60 bis 250 Mikrometer aufweist.

9. Knochenersatzmaterial nach einem der Ansprüche 5 bis 8, das mikroporös, vorzugsweise mit einer offenen Porosität ist.

10. Knochenersatzmaterial nach Anspruch 9, das Mikroporen mit einem mittleren Porendurchmesser von weniger als 10 Mikrometer aufweist, vorzugsweise von weniger als 1 Mikrometer, vorzugsweise von mindestens 5 Nanometer aufweist.

11. Knochenersatzmaterial nach einem der Ansprüche 5 bis 10, mit einer bimodalen Porosität mit Makroporen und Mikroporen.

12. Knochenersatzmaterial nach einem der Ansprüche 5 bis 11, bestehend aus tetragonalem, polykristallinem Zirkondioxid (TZP).

13. Knochenersatzmaterial nach einem der Ansprüche 5 bis 12, bei dem die Porosität im Bereich von 0,1 bis 90 % liegt, vorzugsweise im Bereich von 1 bis 50 %, weiter bevorzugt im Bereich von 5 bis 30 %.

14. Knochenersatzmaterial nach einem der Ansprüche 5 bis 13, in Blockform, vorzugsweise makroporös mit offener Porosität.

15. Knochenersatzmaterial nach einem der Ansprüche 5 bis 14, bei dem die Oberfläche durch eine Ätzbehandlung aufgeraut ist.
